# EUROPEAN PATENT SPECIFICATION

(11) **EP 4 524 572 B1**
(45) Date of publication and mention of the grant of the patent: **28.10.2026**
(21) Application number: 25153299.0
(22) Date of filing: 01.02.2022
(51) Int. Cl.: G01N 33/68, G01N 33/57515

(54) **METHOD FOR DETERMINING WHETHER A SUBJECT IS AT RISK OF DYING FROM BREAST CANCER OR PROSTATE CANCER**
VERFAHREN ZUR BESTIMMUNG DES RISIKOS EINES SUBJEKTS ZUM STERBEN AN BRUSTKREBS ODER PROSTATAKREBS
PROCÉDÉ POUR DÉTERMINER SI UN SUJET PRÉSENTE UN RISQUE DE MOURIR DU CANCER DU SEIN OU DU CANCER DE LA PROSTATE

(30) Priority: 01.02.2021 FI 20215110
(43) Date of publication of application: 19.03.2025
(62) Document number(s) of the earlier application(s) in accordance with Art. 76 EPC: 22703009.5 / 4 285 123
(73) Proprietor: NIGHTINGALE HEALTH OYJ, 00300 Helsinki (FI)
(72) Inventor: Julkunen, Heli, 00300 Helsinki (FI); Würtz, Peter, 00300 Helsinki (FI)
(74) Representative: Papula Oy

(56) References cited:
- JOBARD ELODIE ET AL: "A serum nuclear magnetic resonance-based metabolomic signature of advanced metastatic human breast cancer", CANCER LETTERS, vol. 343, no. 1, 14 September 2013 (2013-09-14), US, pages 33 - 41, XP093082767, ISSN: 0304-3835, DOI: 10.1016/j.canlet.2013.09.011
- SUMAN SHANKAR ET AL: "Metabolic fingerprinting in breast cancer stages through1H NMR spectroscopy-based metabolomic analysis of plasma", JOURNAL OF PHARMACEUTICAL AND BIOMEDICAL ANALYSIS, ELSEVIER B.V, AMSTERDAM, NL, vol. 160, 18 July 2018 (2018-07-18), pages 38 - 45, XP085460842, ISSN: 0731-7085, DOI: 10.1016/J.JPBA.2018.07.024
- FUERTES-MARTÍN ET AL: "Title: Human Serum/Plasma Glycoprotein Analysis by 1H-NMR, an Emerging Method of Inflammatory Assessment", JOURNAL OF CLINICAL MEDICINE, vol. 9, no. 2, 27 January 2020 (2020-01-27), pages 354, XP055920224, DOI: 10.3390/jcm9020354
- DUPREZ DANIEL A ET AL: "Comparison of the Predictive Value of GlycA and Other Biomarkers of Inflammation for Total Death, Incident Cardiovascular Events, Noncardiovascular and Noncancer Inflammatory-Related Events, and Total Cancer Events", vol. 62, no. 7, 1 July 2016 (2016-07-01), US, pages 1020 - 1031, XP093082770, ISSN: 0009-9147, Retrieved from the Internet <URL:http://academic.oup.com/clinchem/article-pdf/62/7/1020/32644709/clinchem1020.pdf> DOI: 10.1373/clinchem.2016.255828

## Description

### TECHNICAL FIELD

The present disclosure relates generally to methods for determining whether a subject is at risk of dying from breast cancer.

### BACKGROUND

Breast and prostate cancers are common cancers and frequent causes of cancer related deaths among women and men, respectively. They also place a great strain on the health care system and have a significant social and financial impact on the affected patients and their families. Fortunately, due to the improved treatment options, these cancers are considered potentially curable when detected at an early stage and it is thus possible to influence the disease burden.

Prediction of breast cancer and prostate cancer survival and an accurate identification of individuals at high risk of dying from these cancers later in life may be important to support early treatment and prevent the development of more severe presentations of these diseases. Various biomarkers may be useful for predicting breast and prostate cancer survival and/or whether an individual person is at an elevated risk of dying from these cancers in the future. Such biomarkers may be measured from various biological samples, for example from blood samples or related biological fluids. Biomarkers predictive of the severe presentations of and risk of mortality from these cancers could support targeted screening, earlier diagnosis and treatment, including guidance for deciding on prophylactic mastectomy. Such a method could have utility both for screening general population without known diagnosis of breast or prostate cancer, as well as for predicting the prognosis and survival among individuals diagnosed with breast cancer or prostate cancer to guide treatment options.

Jobard et al., Cancer Letters 2014, 343, 33-41 describes a serum nuclear magnetic resonance-based metabolomic signature of advanced metastatic human breast cancer.

Suman et al., Journal of Pharmaceutical and Biomedical Analysis 2018, 160, 38-45 describes metabolic fingerprinting in breast cancer stages through ¹H NMR spectroscopy-based metabolomic analysis of plasma.

Fuertes-Martin et al., Journal of Clinical Medicine 2020, 9, 354 describes human serum/plasma glycoprotein analysis by ¹H-NMR.

Duprez et al., Clinical Chemistry 2016, 62:7, 1020-1031 describes a comparison of the predictive value of GlycA and other biomarkers of inflammation for total death, incident cardiovascular events, noncardiovascular and noncancer inflammatory-related events, and total cancer events.

### SUMMARY

A method for determining whether a subject is at risk of dying from breast cancer is disclosed. The method may comprise determining in a biological sample obtained from the subject a quantitative value of at least one biomarker of the following:
- glycoprotein acetyls,
- a ratio of docosahexaenoic acid to total fatty acids,
- a ratio of linoleic acid to total fatty acids,
- a ratio of monounsaturated fatty acids and/or of oleic acid to total fatty acids,
- a ratio of omega-3 fatty acids to total fatty acids,
- a ratio of omega-6 fatty acids to total fatty acids,
- a ratio of saturated fatty acids to total fatty acids,
- fatty acid degree of unsaturation,
- histidine; and

comparing the quantitative value(s) of the at least one biomarker to a control sample or to a control value;
wherein an increase or a decrease in the quantitative value(s) of at least one biomarker, when compared to the control sample or to the control value, is/are indicative of the subject having an increased risk of dying from breast cancer.

### BRIEF DESCRIPTION OF THE DRAWINGS

The accompanying drawings, which are included to provide a further understanding of the embodiments and constitute a part of this specification, illustrate various embodiments. In the drawings:

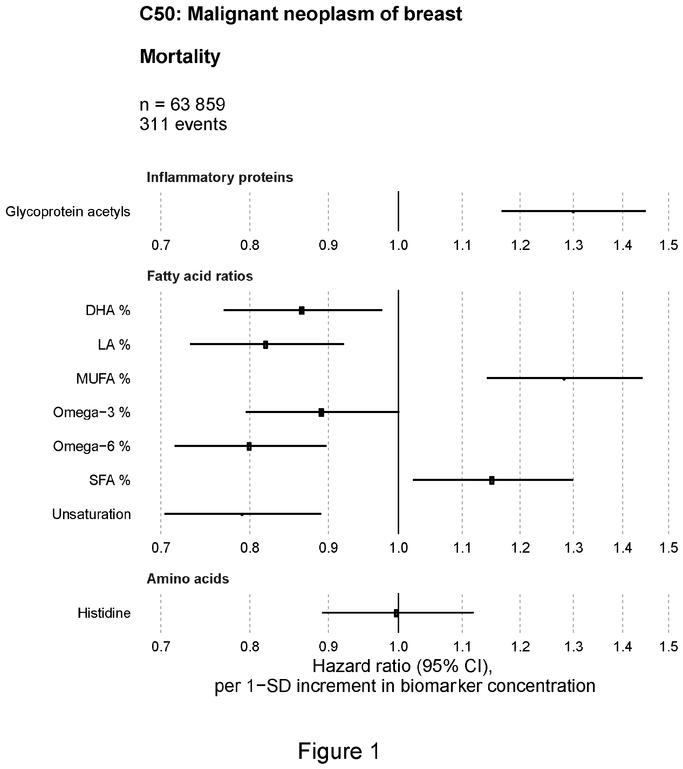

**Figure 1** shows the relation of baseline concentrations of 9 blood biomarkers to breast cancer mortality (malignant neoplasm of breast, ICD-10 diagnosis code C50 in death records) in a general population screening setting (such as along with a mammography test), when the biomarker concentrations are analysed in absolute concentrations scaled to standard deviations of the study population.

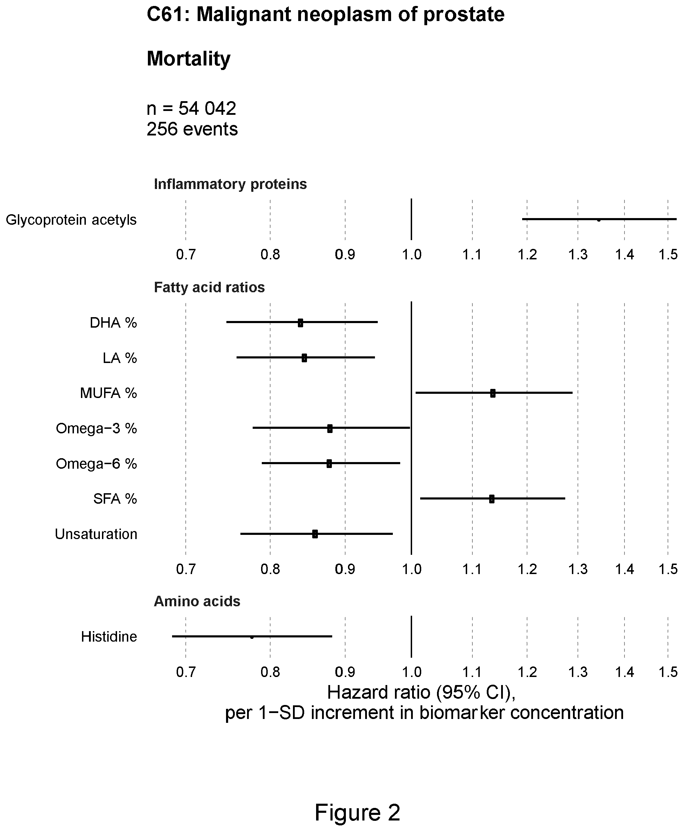

**Figure 2** shows the relation of baseline concentrations of 9 blood biomarkers to prostate cancer mortality (malignant neoplasm of prostate, ICD-10 diagnosis code C51 in death records) in a general population screening setting (such as in conjuction with a prostate-specific antigen (PSA) test), when the biomarker concentrations are analysed in absolute concentrations scaled to standard deviations of the study population.

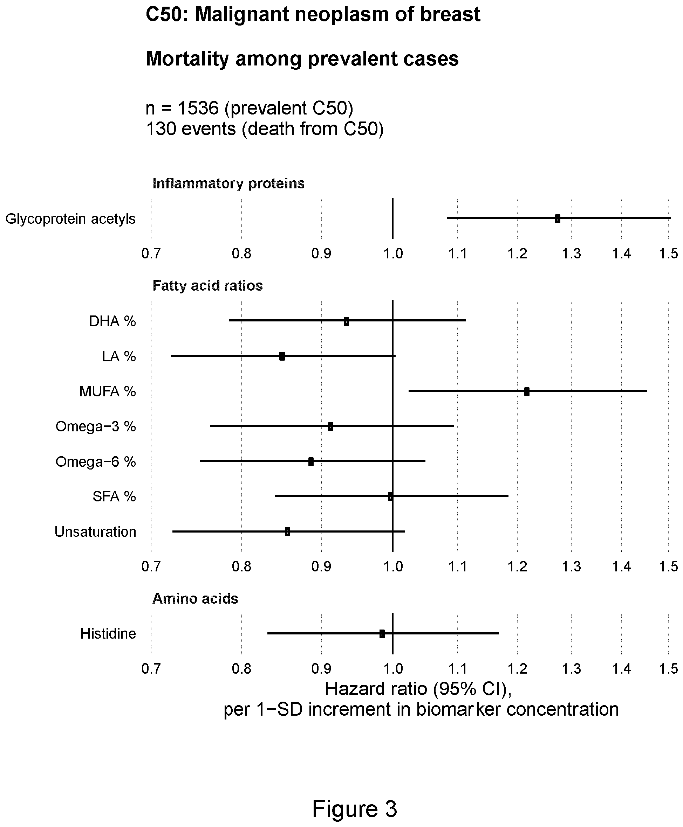

**Figure 3** shows the relation of baseline concentrations of 9 blood biomarkers to breast cancer survival, analysed as breast cancer mortality (malignant neoplasm of breast, ICD-10 diagnosis code C50 in death records) among females with an already diagnosed prevalent breast cancer at the time of the blood sampling (ICD-10 diagnosis code C50 in hospital records), when the biomarker concentrations are analysed in absolute concentrations scaled to standard deviations of the study population.

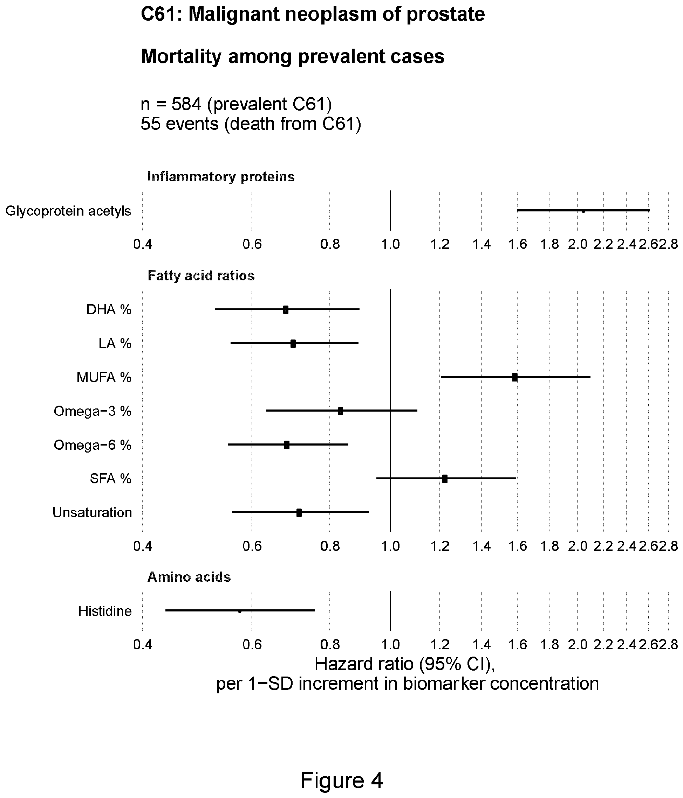

**Figure 4** shows the relation of baseline concentrations of 9 blood biomarkers to prostate cancer survival, analysed as prostate cancer mortality (malignant neoplasm of prostate, ICD-10 diagnosis code C61 in death records) among males with an already diagnosed prevalent prostate cancer at the time of the blood sampling (ICD-10 diagnosis code C61 in hospital records), when the biomarker concentrations are analysed in absolute concentrations scaled to standard deviations of the study population.

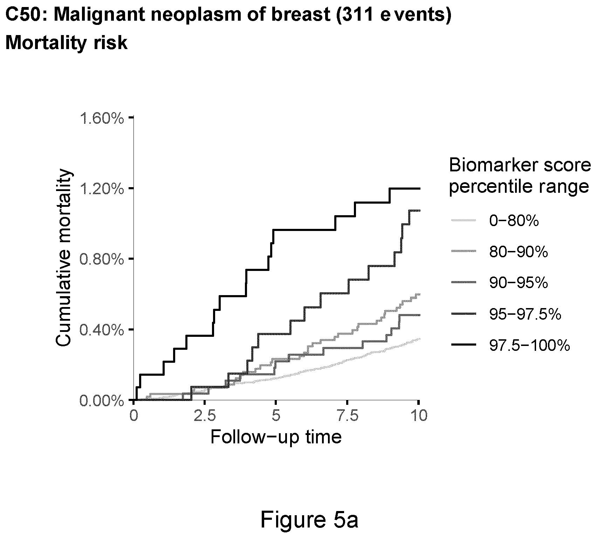

**Figure 5a** shows an intended use case for a multibiomarker score to predict breast cancer mortality risk in a general population screening setting (such as along with a mammography test).

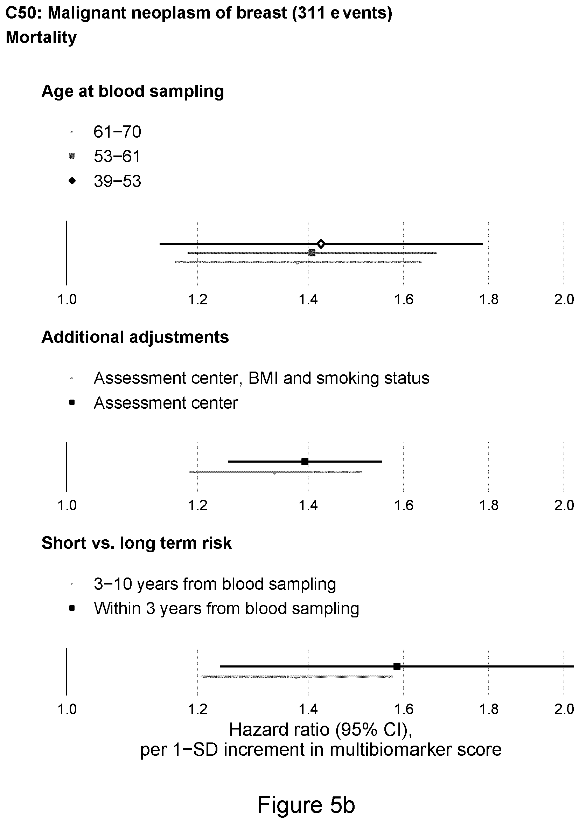

**Figure 5b** shows that the prediction of the risk for breast cancer mortality works effectively for people with different demographics and risk factor profiles, with substantially stronger results for short term risk prediction.

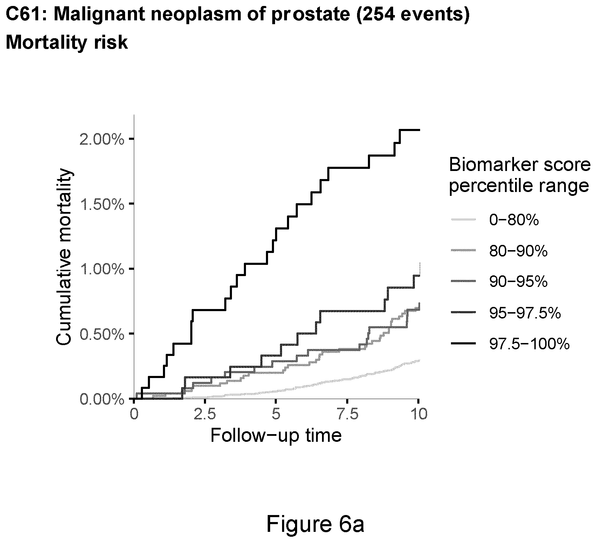

**Figure 6a** shows an intended use case for a multibiomarker score to predict prostate cancer mortality risk in a general population screening setting (such as along with a prostate-specific antigen (PSA) test).

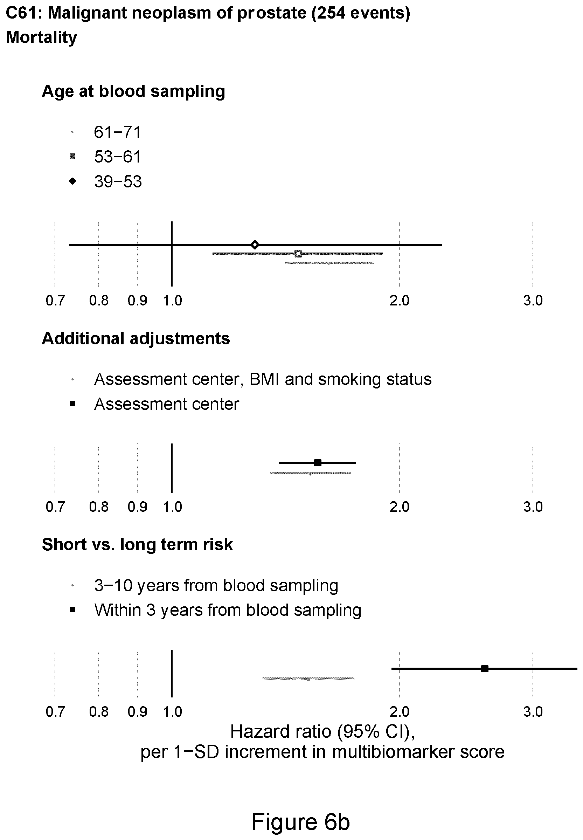

**Figure 6b** shows that the prediction of the risk for prostate cancer mortality works effectively for people with different demographics and risk factor profiles, with substantially stronger results for short term risk prediction.

### DETAILED DESCRIPTION

A method for determining whether a subject is at risk of dying from breast cancer is disclosed.

The method comprises determining in a biological sample obtained from the subject a quantitative value of at least one biomarker of the following:
- glycoprotein acetyls,
- a ratio of docosahexaenoic acid to total fatty acids,
- a ratio of linoleic acid to total fatty acids,
- a ratio of monounsaturated fatty acids and/or of oleic acid to total fatty acids,
- a ratio of omega-3 fatty acids to total fatty acids,
- a ratio of omega-6 fatty acids to total fatty acids,
- a ratio of saturated fatty acids to total fatty acids,
- fatty acid degree of unsaturation,
- histidine; and

and comparing the quantitative value(s) of the at least one biomarker to a control sample or to a control value;
wherein an increase or a decrease in the quantitative value(s) of the at least one biomarker, when compared to the control sample or to the control value, is/are indicative of the subject having an increased risk of dying from breast cancer.

The subject is a woman, i.e. female.

The control sample or control value may represent women without a known breast cancer diagnosis, such as in a population screening setting (such as along with a mammography). Alternatively or additionally, the control sample or control value may represent women already diagnosed with breast cancer. This may allow predicting the prognosis of the cancer.

The subject is a woman without known diagnosis of breast cancer.

The subject may be a woman in a general population screening setting, such as a mammography.

Various blood biomarkers may be useful for predicting whether an individual person is at an elevated risk of dying from breast cancer. Such biomarkers may be measured from biological samples, for example from blood samples or related biological fluids.

Biomarkers predictive of the risk for dying from breast cancer could help to enable more effective screening and better targeted preventative treatment, and/or guide for decision on, for instance, prophylactic mastectomy.

The method comprises determining a quantitative value of glycoprotein acetyls.

In an embodiment, the method comprises determining a quantitative value of the ratio of docosahexaenoic acid to total fatty acids.

In an embodiment, the method comprises determining a quantitative value of the ratio of linoleic acid to total fatty acids.

In an embodiment, the method comprises determining a quantitative value of the ratio of monounsaturated fatty acids and/or oleic acid to total fatty acids.

In an embodiment, the method comprises determining a quantitative value of the ratio of omega-3 fatty acids to total fatty acids.

In an embodiment, the method comprises determining a quantitative value of the ratio of omega-6 fatty acids to total fatty acids.

In an embodiment, the method comprises determining a quantitative value of the ratio of saturated fatty acids to total fatty acids.

In an embodiment, the method comprises determining a quantitative value of fatty acid degree of unsaturation.

In an embodiment, the method comprises determining a quantitative value of histidine.

The metabolic biomarker(s) described in this specification have been found to be significantly different, i.e. their quantitative values (such as amount and/or concentration) have been found to be significantly higher or lower, for subjects who later died from breast cancer. The biomarkers may be detected and quantified from blood, serum, or plasma, dry blood spots, or other suitable biological sample, and may be used to predict the prognosis and/or survival, i.e. to determine the risk of dying from breast cancer, either alone or in combination with other biomarkers.

Furthermore, the biomarker(s) may significantly improve the possibility of identifying subjects at risk of dying from breast cancer, even in combination with and/or when accounting for established factors that may currently be used for prediction of the prognosis and/or survival, such as age, family history, smoking status, body mass index (BMI), existing comorbidities, inflammatory protein biomarkers such as C-reactive protein and/or albumin, stage of the cancer and metastasis, genetic variants including BRCA1 and BRCA2 alleles, tumor size, tumor grade, certain traits of the cancer cells and/or response to the treatment. The biomarkers described in this specification, alone or as a risk score (such as a multi-biomarker score), hazard ratio, odds ratio, and/or predicted absolute or relative risk, or in combination with other risk factors and tests, such as mammography, may improve the prediction accuracy. This may include improving prediction accuracy by complementing the predictive information from other risk factors, or by replacing the need for other analyses. The biomarkers or the risk score, hazard ratio, odds ratio, and/or predicted absolute or relative risk according to one or more embodiments described in this specification may thus allow for efficiently assessing the risk for dying from breast cancer, also in conditions in which other risk factor measures are not as feasible.

In an embodiment, the method is a method for determining whether the subject is at risk of dying from breast cancer within (the following) three years.

The method may comprise determining in the biological sample quantitative values of a plurality of the biomarkers, such as two, three, four, five or more biomarkers. For example, the plurality of the biomarkers may comprise 2, 3, 4, 5, 6, 7, 8, 9, or 10 (i.e. at least 2, at least 3, at least 4, at least 5, at least 6, at least 7, at least 8, at least 9, or all of the biomarkers). The term "plurality of the biomarkers" may thus, within this specification, be understood as referring to any number (above one) of the biomarkers. The term "plurality of the biomarkers" may thus be understood as referring to any number (above one) and/or combination or subset of the biomarkers described in this specification. Determining the plurality of the biomarkers may increase the accuracy of the prediction of whether the subject is at risk of dying from breast cancer. In general, it may be that the higher the number of the biomarkers, the more accurate or predictive the method. However, even a single biomarker described in this specification may allow for or assist in determining whether the subject is at risk of dying from breast cancer. The plurality of the biomarkers may be measured from the same biological sample or from separate biological samples and using the same analytical method or different analytical methods. In an embodiment, the plurality of biomarkers may be a panel of a plurality of biomarkers.

In the context of this specification, the wording "comparing the quantitative value(s) of the biomarker(s) to a control sample or to a control value(s)" may be understood, as a skilled person would, as referring to comparing the quantitative value or values of the biomarker or biomarkers, to a control sample or to a control value(s) either individually or as a plurality of biomarkers (e.g. when a risk score is calculated from the quantitative values of a plurality of biomarkers), depending e.g. on whether the quantitative value of a single (individual) biomarker or the quantitative values of a plurality of biomarkers are determined. The control sample or control value may represent women without known breast cancer diagnosis, respectively, for example as in population screening settings (such as along with a mammography), or women already diagnosed with breast cancer to predict the prognosis of the cancer.

In an embodiment, the method may comprise determining in the biological sample obtained from the subject a quantitative value of the following biomarkers:
- glycoprotein acetyls,
- a ratio of docosahexaenoic acid to total fatty acids,
- a ratio of linoleic acid to total fatty acids,
- a ratio of monounsaturated fatty acids and/or of oleic acid to total fatty acids,
- a ratio of omega-3 fatty acids to total fatty acids,
- a ratio of omega-6 fatty acids to total fatty acids,
- a ratio of saturated fatty acids to total fatty acids,
- fatty acid degree of unsaturation,
- histidine; and

comparing the quantitative value(s) of the biomarkers to a control sample or to a control value(s);
wherein an increase or a decrease in the quantitative value(s) of the biomarkers, when compared to the control sample or to the control value, is/are indicative of the subject having an increased risk of dying from breast cancer.

The at least one biomarker comprises or is glycoprotein acetyls. The method may further comprise determining a quantitative value of at least one of the other biomarkers described in this specification.

The subject may be human. The human may be healthy or have an existing disease, such as an existing cancer. Specifically, the human may have an already existing form of breast cancer, and the risk for developing more severe forms of and/or dying from these cancers may be determined and/or calculated.

In the context of this specification, the term "biomarker" may refer to a biomarker, for example a chemical or a molecular marker, that may be found to be associated with a severe course of a disease or the risk of dying from a disease. It does not necessarily refer to a biomarker that would be statistically fully validated as having a specific effectiveness in a clinical setting. The biomarker may be a metabolite, a compound, a lipid, a protein, a moiety, a functional group, a composition, a combination of two or more metabolites and/or compounds, a (measurable or measured) quantity thereof, a ratio or other value derived thereof, or in principle any measurement reflecting a chemical and/or biological component that may be found to be associated with dying from a disease. The biomarkers and any combinations thereof, optionally in combination with further analyses and/or measures, may be used to measure a biological process indicative of the risk for dying from breast cancer.

In the context of this specification, the term "dying from breast cancer" may be understood as referring to death caused by breast cancer. In other words, the subject having an increased risk of dying from breast cancer may be understood as the subject having an increased risk of developing a fatal breast cancer, i.e. death from or caused by the breast cancer, e.g. either among individuals without known diagnosis of these cancers (as in population screening settings) to reflect the risk of developing a fatal form of these cancers later in life or among individuals with an existing diagnosis of these cancers to reflect the survival after diagnosis.

The subject is a woman without known diagnosis of breast cancer. The subject may, additionally or alternatively, be a woman or man in a general population screening setting, such as a mammography.

Breast and/or prostate cancers described in this specification may be classified as follows. "ICD-10" may be understood as referring to the International Statistical Classification of Diseases and Related Health Problems 10th Revision (ICD-10) - WHO Version for 2019. Similar cancers classified or diagnosed by other disease classification on systems than ICD-10, such as ICD-9 or ICD-11, may also apply.

Breast cancers may be understood as referring to diseases classified within the 3-character ICD-10 diagnoses for breast cancers (C50: malignant neoplasm of breast).

In an embodiment, breast cancer is a specific disease, such as defined by ICD-10 3-character code diagnoses.

In an embodiment, breast cancer is the following ICD-10 3-character diagnosis:
- C50: Malignant neoplasm of breast

In an embodiment, the death from breast cancer may comprise or be death from breast cancer, such as a death from a disease denoted by any one of the ICD-10 codes listed above.

In an embodiment, the breast cancer comprises or is malignant neoplasm of breast.

The method may further comprise determining whether the subject is at risk of dying from breast cancer using a risk score, hazard ratio, odds ratio, and/or predicted absolute or relative risk calculated on the basis of the quantitative value(s) of the at least one biomarker or of the plurality of the biomarkers.

An increase or a decrease in the risk score, hazard ratio, and/or predicted absolute risk and/or relative risk may be indicative of the subject having an increased risk of dying from breast cancer. The prediction may be applied, for instance, among women without known breast cancer diagnosis, respectively, as in a general population screening setting, such as along with a mammography, or among individuals already diagnosed with breast cancer to predict the survival.

The risk score and/or hazard ratio and/or predicted absolute risk or relative risk may be calculated based on any plurality, combination or subset of biomarkers described in this specification.

The risk score and/or hazard ratio and/or predicted absolute risk or relative risk may be calculated **e.g.** as shown in the Examples below. For example, the plurality of biomarkers measured using a suitable method, for example with nuclear magnetic resonance (NMR) spectroscopy, may be combined using regression algorithms and multivariate analyses and/or using machine learning analysis. Before regression analysis or machine learning, any missing values in the biomarkers may be imputed with the mean value of each biomarker for the dataset. A number of biomarkers, for example five, that may be considered most associated with death from the disease may be selected for the use in the prediction model. Other modelling approaches may be used to calculate a risk score and/or hazard ratio and/or predicted absolute risk or relative risk based on a combination or subset of individual biomarkers, i.e. a plurality of the biomarkers.

The risk score may be calculated e.g. as a weighted sum of individual biomarkers, i.e. a plurality of the biomarkers. The weighted sum may be e.g. in the form of a multi-biomarker score defined as ${\sum }_{i} \left〚{β}_{i}*{c}_{i}\right〛 + {β}_{0}$; where *i* is the index of summation over individual biomarkers, β*ᵢ* is the weighted coefficient attributed to biomarker *i, cᵢ* is the blood concentration of biomarker *i,* and β*₀* is an intercept term.

For example, the risk score can be defined as: β₁*concentration(glycoprotein acetyls) + β₂* concentration(monounsaturated fatty acid ratio to total fatty acids) + β₃* concentration (histidine) + β₀, where β₁, β₂, β₃ are multipliers for each biomarker according to the association magnitude with risk of dying from breast cancer and β₀ is an intercept term. As a skilled person will understand, the biomarkers mentioned in this example may be replaced by any other biomarker(s) described in this specification. In general, the more biomarkers are included in the risk score, the stronger the predictive performance may become. When additional biomarkers are included in the risk score, the β*i* weights may change for all biomarkers according to the optimal combination for the prediction of death from breast cancer.

The risk score, hazard ratio, odds ratio, and/or predicted relative risk and/or absolute risk may be calculated on the basis of at least one further measure, for example a characteristic of the subject. Such characteristics may be determined prior to, simultaneously, or after the biological sample is obtained from the subject. As a skilled person will understand, some of the characteristics may be information collected e.g. using a questionnaire or clinical data collected earlier. Some of the characteristics may be determined (or may have been determined) by biochemical or clinical diagnostic measurements and/or medical diagnosis. Such characteristics could include, for example, one or more of age, height, weight, body mass index, race or ethnic group, smoking, family history of cancers and/or information from mammography.

The risk prediction for death from breast cancer guided based on one or more of the biomarkers can be used to guide treatment and preventative efforts for a severe course of the cancer, such as alcohol and smoking awareness, healthy diet, physical activity, clinical screening frequency and/or treatment decisions. For example, the information of the risk for death from breast cancer can be used for guiding frequency of cancer screenings, decision on prophylactic mastectomy and/or treatment with, for instance, aspirin and other non-steroidal antiinflammatory drugs (NSAIDs), and/or selective estrogen receptor modulators (SERMs), such as tamoxifen and/or raloxifene.

In the context of this specification, the term "glycoprotein acetyls", "glycoprotein acetylation", or "GlycA" may refer to the abundance of circulating glycated proteins, and/or to a nuclear magnetic resonance spectroscopy (NMR) signal that represents the abundance of circulating glycated proteins, i.e. N-acetylated glycoproteins. Glycoprotein acetyls may include signals from a plurality of different glycoproteins, including e.g. alpha-1-acid glycoprotein, alpha-1 antitrypsin, haptoglobin, transferrin, and/or alpha-1 antichymotrypsin. In the scientific literature on cardiometabolic biomarkers, the terms "glycoprotein acetyls" or "GlycA" may commonly refer to the NMR signal of circulating glycated proteins (e.g. Ritchie et al, Cell Systems 2015 1(4):293-301; Connelly et al, J Transl Med. 2017;15(1):219). Glycoprotein acetyls and a method for measuring them is described e.g. in Kettunen et al., 2018, Circ Genom Precis Med. 11:e002234 and Soininen et al., 2009, Analyst 134, 1781-1785. There may be benefits of using the NMR signal of glycoprotein acetyls for risk prediction above measurement of the individual proteins contributing to the NMR signal, for instance better analytical accuracy and stability over time, as well as lower costs of the measurement and the possibility to measure the NMR signal simultaneously with many other biomarkers.

In the context of this specification, the term "omega-3 fatty acids" may refer to total omega-3 fatty acids, i.e. the total omega-3 fatty acid amounts and/or concentrations, i.e. the sum of different omega-3 fatty acids. Omega-3 fatty acids are polyunsaturated fatty acids. In omega-3 fatty acids, the last double bond in the fatty acid chain is the third bond counting from the methyl end. Docosahexaenoic acid is an example of an omega-3 fatty acid.

In the context of this specification, the term "omega-6 fatty acids" may refer to total omega-6 fatty acids, i.e. the total omega-6 fatty acid amounts and/or concentrations, i.e. the sum of the amounts and/or concentrations of different omega-6 fatty acids. Omega-6 fatty acids are polyunsaturated fatty acids. In omega-6 fatty acids, the last double bond in the fatty acid chain is the sixth bond counting from the methyl end.

In one embodiment, the omega-6 fatty acid may be linoleic acid. Linoleic acid (18:2ω-6) is the most abundant type of omega-6 fatty acids, and may therefore be considered as a good approximation for total omega-6 fatty acids for risk prediction of dying from breast cancer.

In the context of this specification, the term "monounsaturated fatty acids" (MUFAs) may refer to total monounsaturated fatty acids, i.e. the total MUFA amounts and/or concentrations. Monounsaturated fatty acids may, alternatively, refer to oleic acid, which is the most abundant monounsaturated fatty acid in human serum. Monounsaturated fatty acids have one double bond in their fatty acid chain. The monounsaturated fatty acids may include omega-9 and omega-7 fatty acids. Oleic acid (18:1ω-9), palmitoleic acid (16:1ω-7) and cis-vaccenic acid (18:1ω-7) are examples of common monounsaturated fatty acids in human serum.

In one embodiment, the monounsaturated fatty acid may be oleic acid. Oleic acid is the most abundant monounsaturated fatty acid, and may therefore be considered as a good approximation for total monounsaturated fatty acids for risk prediction of dying from breast cancer.

In the context of this specification, the term "saturated fatty acids" (SFAs) may refer to total saturated fatty acids. Saturated fatty acids may be or comprise fatty acids which have no double bonds in their structure. Palmitic acid (16:0) and stearic acid (18:0) are examples of abundant SFAs in human serum.

For all fatty acid measures, including omega-6, docosahexaenoic acid, linoleic acid, monounsaturated fatty acids and/or saturated fatty acids, the fatty acid measures may include blood (or serum/plasma) free fatty acids, bound fatty acids and esterified fatty acids. Esterified fatty acids may, for example, be esterified to glycerol as in triglycerides, diglycerides, monoglycerides, or phosphoglycerides, or to cholesterol as in cholesterol esters.

In the context of this specification, the term "fatty acid degree of unsaturation" or "unsaturation" may be understood as referring to the number of double bonds in total fatty acids, for example the average number of double bonds in total fatty acids.

In the context of this specification, the term "histidine" may refer to the histidine amino acid, for example in blood, plasma or serum or related biofluids.

In the context of this specification, the term "quantitative value" may refer to any quantitative value characterizing the amount and/or concentration of a biomarker. For example, it may be the amount or concentration of the biomarker in the biological sample, or it may be a signal derived from nuclear magnetic resonance spectroscopy (NMR) or other method suitable for detecting the biomarker in a quantitative manner. Such a signal may be indicative of or may correlate with the amount or concentration of the biomarker. It may also be a quantitative value calculated from one or more signals derived from NMR measurements or from other measurements. Quantitative values may, additionally or alternatively, be measured using a variety of techniques. Such methods may include mass spectrometry (MS), gas chromatography combined with MS, high performance liquid chromatography alone or combined with MS, immunoturbidimetric measurements, ultracentrifugation, ion mobility, enzymatic analyses, colorimetric or fluorometric analyses, immunoblot analysis, immunohistochemical methods (e.g. *in situ* methods based on antibody detection of metabolites), and immunoassays (e.g. ELISA). Examples of various methods are set out below. The method used to determine the quantitative value(s) in the subject may be the same method that is used to determine the quantitative value(s) in a control subject/control subjects or in a control sample/control samples.

The quantitative value, or the initial quantitative value, of the at least one biomarker, or the plurality of the biomarkers, may be measured using nuclear magnetic resonance (NMR) spectroscopy, for example ¹H-NMR. The at least one additional biomarker, or the plurality of the additional biomarkers, may also be measured using NMR. NMR may provide a particularly efficient and fast way to measure biomarkers, including a large number of biomarkers simultaneously, and can provide quantitative values for them. NMR also typically requires very little sample pre-treatment or preparation. The biomarkers measured with NMR can effectively be measured for large amounts of samples using an assay for blood (serum or plasma) NMR metabolomics previously published by Soininen et al., 2015, Circulation: Cardiovascular Genetics 8, 212-206 (DOI: 10.1161/CIRCGENETICS.114.000216); Soininen et al., 2009, Analyst 134, 1781-1785; and Würtz et al., 2017, American Journal of Epidemiology 186 (9), 1084-1096 (DOI: 10.1093/aje/kwx016). This provides data on 250 biomarkers per sample as described in detail in the above scientific papers.

In an embodiment, the (initial) quantitative value(s) of the at least one biomarker is/are measured using nuclear magnetic resonance spectroscopy.

However, quantitative values for various biomarkers described in this specification may also be performed by techniques other than NMR. For example, mass spectrometry (MS), enzymatic methods, antibody-based detection methods, or other biochemical or chemical methods may be contemplated, depending on the biomarker.

For example, glycoprotein acetyls can be measured or approximated by immunoturbidimetric measurements of alpha-1-acid glycoprotein, haptoglobin, alpha-1-antitrypsin, and transferrin (e.g. as described in Ritchie et al., 2015, Cell Syst. 28;1(4):293-301).

E.g. monounsaturated fatty acids, saturated fatty acids, and omega-6 fatty acids can be quantified (i.e. their quantitative values may be determined) by serum total fatty acid composition using gas chromatography (for example, as described in Jula et al., 2005, Arterioscler Thromb Vasc Biol 25, 2152-2159).

In the context of this specification, the term "sample" or "biological sample" may refer to any biological sample obtained from a subject or a group or population of subjects. The sample may be fresh, frozen, or dry.

The biological sample may comprise or be, for example, a blood sample, a plasma sample, a serum sample, or a sample or fraction derived therefrom. The biological sample may be, for example, a fasting blood sample, a fasting plasma sample, a fasting serum sample, or a fraction obtainable therefrom. However, the biological sample does not necessarily have to be a fasting sample. The blood sample may be a venous blood sample.

The blood sample may be a dry blood sample. The dry blood sample may be a dried whole blood sample, a dried plasma sample, a dried serum sample, or a dried sample derived therefrom.

The method may comprise obtaining the biological sample from the subject prior to determining the quantitative value of the at least one biomarker. Taking a blood sample or a tissue sample of a subject or patient is a part of normal clinical practice. The collected blood or tissue sample can be prepared and serum or plasma can be separated using techniques well known to a skilled person. Methods for separating one or more fractions from biological samples, such as blood samples or tissue samples, are also available to a skilled person. The term "fraction" may, in the context of this specification, also refer to a portion or a component of the biological sample separated according to one or more physical properties, for instance solubility, hydrophilicity or hydrophobicity, density, or molecular size.

In the context of this specification, the term "control sample" may refer to a sample obtained from a subject and known not to suffer from the disease or condition or not being at risk of having or developing the disease or condition. In an embodiment, the control sample may be a biological sample from a subject, such as a woman, or a generalized population of women without known positive diagnosis of breast cancer, respectively. In other words, the control sample may be a biological sample from a woman or a generalized population of women who has/have not been (previously) diagnosed with breast cancer. Alternatively, in an embodiment, the control population may be a sample of women already diagnosed with breast cancer. It may be compared to samples developing and/or dying from these cancers to predict the mortality risk. The control sample may be matched. The term "control value" may be understood as a value obtainable from the control sample and/or a quantitative value derivable therefrom. In an embodiment, the control value may be a value derivable from one or more women or a generalized population of women without known positive diagnosis of breast cancer, respectively. For example, it may be possible to calculate a threshold value from control samples and/or control values, above or below which the risk of dying from the cancer is elevated. In other words, a value higher or lower (depending on the biomarker, risk score, hazard ratio, and/or predicted absolute risk or relative risk) than the threshold value may be indicative of the subject having an increased risk of dying from the cancer. The risk score, hazard ratio, odds ratio, and/or predicted absolute or relative risk may be used alone or in combination with other risk factors and measures, such as information from mammography and/or family history.

An increase or a decrease in the quantitative value(s) of the at least one biomarker, or the plurality of the biomarkers, when compared to the control sample or to the control value, may be indicative of the subject having an increased risk of dying from the cancer. Whether an increase or a decrease is indicative of the subject having an increased risk of dying from the cancer, may depend on the biomarker.

A 1.2-fold, 1.5-fold, or for example 2-fold, or 3-fold, increase or a decrease in the quantitative value(s) of the at least one biomarker (or in an individual biomarker of the plurality of the biomarkers) when compared to the control sample or to the control value, may be indicative of the subject having an increased risk of dying from the cancer.

In an embodiment, an increase in the quantitative value of glycoprotein acetyls, when compared to the control sample or to the control value, may be indicative of the subject having an increased risk of dying from breast cancer.

In an embodiment, a decrease in the quantitative value of docosahexaenoic acid and/or the ratio of docosahexaenoic acid to total fatty acids, when compared to the control sample or to the control value, may be indicative of the subject having an increased risk of dying from breast cancer.

In an embodiment, a decrease in the quantitative value of linoleic acid and/or the ratio linoleic acid to total fatty acids, when compared to the control sample or to the control value, may be indicative of the subject having an increased risk of dying from breast cancer.

In an embodiment, an increase in the quantitative value of monounsaturated fatty acids and/or oleic acid and/or the ratio of monounsaturated fatty acids and/or oleic acid to total fatty acids, when compared to the control sample or to the control value, may be indicative of the subject having an increased risk of dying from breast cancer.

In an embodiment, a decrease in the quantitative value of omega-3 fatty acids and/or the ratio of omega-3 fatty acids to total fatty acids, when compared to the control sample or to the control value, may be indicative of the subject having an increased risk of dying from breast cancer.

In an embodiment, a decrease in the quantitative value of omega-6 fatty acids and/or the ratio of omega-6 fatty acids to total fatty acids, when compared to the control sample or to the control value, may be indicative of the subject having an increased risk of dying from breast cancer.

In an embodiment, an increase in the quantitative value of saturated fatty acids and/or the ratio of saturated fatty acids to total fatty acids, when compared to the control sample or to the control value, may be indicative of the subject having an increased risk of dying from breast cancer.

In an embodiment, a decrease in the quantitative value of fatty acid degree of unsaturation, when compared to the control sample or to the control value, may be indicative of the subject having an increased risk of dying from breast cancer.

In an embodiment, a decrease in the quantitative value of histidine, when compared to the control sample or to the control value, may be indicative of the subject having an increased risk of dying from breast cancer.

In an embodiment, a risk score defined as β₀ + β₁* concentration (glycoprotein acetyls) + β₂* concentration (fatty acid measure), where β₀ is an intercept term, β₁ is the weighted coefficient attributed to the concentration of glycoprotein acetyls, β₂ is the weighted coefficient attributed to the fatty acid measure, may be indicative of the subject having an increased risk of dying from breast cancer. The fatty acid measure may be one or more of the following fatty acid measures: the ratio of docosahexaenoic acid to total fatty acids, the ratio of linoleic acid to total fatty acids, the ratio of monounsaturated fatty acids and/or of oleic acid to total fatty acids, the ratio of omega-3 fatty acids to total fatty acids, the ratio of omega-6 fatty acids to total fatty acids, the ratio of saturated fatty acids to total fatty acids, fatty acid degree of unsaturation.

In an embodiment, a risk score defined as β₀ + β₁* concentration (glycoprotein acetyls) + β₂* concentration (histidine) + β₃* concentration (fatty acid measure), where β₀ is an intercept term, β₁ is the weighted coefficient attributed to the concentration of glycoprotein acetyls, β₂ is the weighted coefficient attributed to the concentration of histidine, and β₃ is the weighted coefficient attributed to the concentration of the fatty acid measure may be indicative of the subject having an increased risk of dying from breast cancer. The fatty acid measure may be one or more of the following fatty measures: the ratio of docosahexaenoic acid to total fatty acids, the ratio of linoleic acid to total fatty acids, the ratio of monounsaturated fatty acids and/or of oleic acid to total fatty acids, the ratio of omega-3 fatty acids to total fatty acids, the ratio of omega-6 fatty acids to total fatty acids, the ratio of saturated fatty acids to total fatty acids, fatty acid degree of unsaturation.

The term "combination" may, at least in some embodiments, be understood such that the method comprises using a risk score, hazard ratio, odds ratio, and/or predicted absolute risk or relative risk calculated on the basis of the quantitative value(s) of the biomarkers. For example, if quantitative values of both glycoprotein acetyls and a fatty acid measure are determined, the quantitative values of both biomarkers may be compared to the control sample or the control value separately, or a risk score, hazard ratio, odds ratio, and/or predicted absolute risk or relative risk calculated on the basis of the quantitative value(s) of both the biomarkers, and the risk score, hazard ratio, odds ratio, and/or predicted absolute risk or relative risk may be compared to the control sample or the control value.

In an embodiment, the method may comprise determining in the biological sample obtained from the subject a quantitative value of the following biomarkers:
- glycoprotein acetyls,
- at least one fatty acid measure(s) of the following: the ratio of docosahexaenoic acid to total fatty acids, the ratio of linoleic acid to total fatty acids, the ratio of monounsaturated fatty acids and/or of oleic acid to total fatty acids, the ratio of omega-3 fatty acids to total fatty acids, the ratio of omega-6 fatty acids to total fatty acids, the ratio of saturated fatty acids to total fatty acids, fatty acid degree of unsaturation; and

comparing the quantitative value(s) of the biomarkers and/or a combination thereof to a control sample or to a control value(s);
wherein an increase or a decrease in the quantitative value(s) of the biomarkers and/or the combination thereof, when compared to the control sample or to the control value, is/are indicative of the subject having an increased risk of dying from breast cancer. An increase in the quantitative value of glycoprotein acetyls and a decrease in the quantitative value of the ratio of docosahexaenoic and/or linoleic acid and/or omega-3 fatty acids and/or omega-6 fatty acids to total fatty acids and/or fatty acid degree of unsaturation, and/or an increase in the quantitative value of the ratio of monounsaturated fatty acids and/or saturated fatty acids to total fatty acids, when compared to the control sample or to the control value, may be indicative of the subject having an increased risk of dying from breast cancer.

In an embodiment, the method may comprise determining in the biological sample obtained from the subject a quantitative value of the following biomarkers:
- glycoprotein acetyls,
- histidine,
- at least one fatty acid measure(s) of the following: the ratio of docosahexaenoic acid to total fatty acids, the ratio of linoleic acid to total fatty acids, the ratio of monounsaturated fatty acids and/or of oleic acid to total fatty acids, the ratio of omega-3 fatty acids to total fatty acids, the ratio of omega-6 fatty acids to total fatty acids, the ratio of saturated fatty acids to total fatty acids, fatty acid degree of unsaturation; and

comparing the quantitative value(s) of the biomarkers and/or a combination thereof to a control sample or to a control value(s);
wherein an increase or a decrease in the quantitative value(s) of the biomarkers and/or the combination thereof, when compared to the control sample or to the control value, is/are indicative of the subject having an increased risk of dying from breast cancer. An increase in the quantitative value of glycoprotein acetyls, a decrease in the quantitative value of histidine, and a decrease in the quantitative value of the ratio of docosahexaenoic and/or linoleic acid and/or omega-3 fatty acids and/or omega-6 fatty acids to total fatty acids and/or fatty acid degree of unsaturation, and/or an increase in the quantitative value of the ratio of monounsaturated fatty acids and/or saturated fatty acids and/or to total fatty acids, when compared to the control sample or to the control value, may be indicative of the subject having an increased risk of dying from breast cancer.

### EXAMPLES

Reference will now be made in detail to various embodiments, an example of which is illustrated in the accompanying drawings. The description below discloses some embodiments in such a detail that a person skilled in the art is able to utilize the embodiments based on the disclosure. Not all steps or features of the embodiments are discussed in detail, as many of the steps or features will be obvious for the person skilled in the art based on this specification.

### Abbreviations used in the Figures:

DHA %: Ratio of docosahexaenoic acid to total fatty acids LA%: Ratio of linoleic acid to total fatty acids
MUFA %: Ratio of monounsaturated fatty acids to total fatty acids
Omega-3 %: Ratio of omega-3 fatty acids to total fatty acids
Omega-6 %: Ratio of omega-6 fatty acids to total fatty acids
SFA %: Ratio of saturated fatty acids to total fatty acids
Unsaturation: Fatty acid degree of unsaturation
CI: confidence interval
SD: standard deviation
BMI: Body mass index

### EXAMPLE 1

Biomarker measures quantified by NMR spectroscopy were investigated as to whether they could be predictive of dying from breast cancer or prostate cancer. All analyses were conducted based on the UK Biobank, with approximately 115 000 study participants with blood biomarker data from NMR spectroscopy available.

### Study population

Details of the design of the UK Biobank have been reported by Sudlow et al 2015, PLoS Med. 2015;12(3):e1001779. Briefly, UK Biobank recruited 502 639 participants aged 37-73 years in 22 assessment centres across the UK. All participants provided written informed consent and ethical approval was obtained from the North West Multi-Center Research Ethics Committee. Blood samples were drawn at baseline between 2007 and 2010. No selection criteria were applied to the sampling.

### Biomarker profiling

From the entire UK Biobank population, a random subset of baseline plasma samples from 118 466 individuals were measured using the Nightingale NMR biomarker platform (Nightingale Health Ltd, Finland). This blood analysis method provides simultaneous quantification of many blood biomarkers, including lipoprotein lipids, circulating fatty acids, and various low-molecular weight metabolites including amino acids, ketone bodies and gluconeogenesis-related metabolites in molar concentration units. Technical details and epidemiological applications have been reviewed (Soininen et al 2015, Circ Cardiovasc Genet; 2015;8:192-206; Würtz et al 2017, Am J Epidemiol 2017;186:1084-1096). Values outside four interquartile ranges from median were considered as outliers and excluded.

### Epidemiological analyses of biomarker relations with the risk of dying from breast cancer or prostate cancer

The blood biomarker associations with the risk of dying from breast cancer or prostate cancer were conducted based on UK Biobank data. Analyses focused on the relation of the biomarkers to the mortality from breast cancer or prostate cancer after the blood samples were collected, to determine if the individual biomarkers associate with the risk of dying from breast cancer or prostate cancer. Examples using multi-biomarker scores, in the form weighted sums of biomarkers, were also explored to demonstrate their effectiveness in various prediction settings.

Information on the disease and mortality events for all study participants were recorded from UK Hospital Episode Statistics data and death registries. Analyses were performed using two approaches: by analyzing the mortality risk among women and men without known positive diagnosis of prostate or breast cancer, respectively, as in general population screening settings (such as along with a mammography or PSA screening test, respectively), as well as among women or men already diagnosed with breast cancer or prostate cancer at the time of the blood sampling, respectively, to predict the prognosis of the cancer. Breast and prostate cancers analyzed here were defined according to the ICD-10 diagnosis codes C50 (malignant neoplasm of breast) and C61 (malignant neoplasm of prostate). The registry-based follow-up was from blood sampling in 2007-2010 through to 2020 (approximately 1 100 000 person-years).

For biomarker association testing, Cox proportional-hazard regression models adjusted for age and UK Biobank assessment centre were used. Results were plotted in magnitudes per standard deviation of each biomarker measure to allow direct comparison of association magnitudes.

### Summary of results

Baseline characteristics of the study population for biomarker analyses are shown in Table 1.

**Table 1: Clinical characteristics of study participants.**

| | |
|---|---|
| **Total number of individuals with blood samples analysed** | 118 456 |

| **Study setting** | Population sample of study volunteers from the UK |
|---|---|
| Percentage of women | 54.1% |
| Age range (years) | 39 - 71 |
| Median age (years) | 58 |
| Median BMI (kg/m²) | 26.8 |
| Follow-up time for disease events after blood sampling | 10-14 years |

| **Breast cancer (C50)** | |
|---|---|
| Number of total breast cancer mortality events | 311 |
| Number of participants with prevalent breast cancer | 1 536 |
| Number of breast cancer mortality events among participants with prevalent breast cancer | 130 |

| **Prostate cancer (C61)** | |
|---|---|
| Number of prostate cancer mortality events | 256 |
| Number of participants with prevalent prostate cancer | 584 |
| Number of prostate cancer mortality events among participants with prevalent prostate cancer | 55 |

**Figure 1** shows the relation of baseline concentrations of 9 blood biomarkers to breast cancer mortality (ICD-10 diagnosis code C50 in death records) in a general population screening setting (such as along with a mammography test), when the biomarker concentrations are analysed in absolute concentrations scaled to standard deviations of the study population. The results are based on statistical analyses of 63 859 females from the UK Biobank, out of whom 311 individuals died from breast cancer during approximately 10 years of follow-up. The analyses were adjusted for age and UK Biobank assessment centre in Cox proportional-hazard regression models. In the forestplots, statistically significant results are displayed as filled points (P<0.001 corresponding to multiple testing correction) and non-significant results are displayed as hollow points. These results demonstrate that the 9 individual biomarkers are predictive of the risk for dying from breast cancer in general population settings.

**Figure 2** shows the relation of baseline concentrations of 9 blood biomarkers to prostate cancer mortality (ICD-10 diagnosis code C61 in death records) in a general population screening setting (such as along with a prostate-specific antigen (PSA) test), when the biomarker concentrations are analysed in absolute concentrations scaled to standard deviations of the study population. The results are based on statistical analyses of 54 042 males from the UK Biobank, out of whom 256 individuals died from prostate cancer during approximately 10 years of follow-up. The analyses were adjusted for age and UK Biobank assessment centre in Cox proportional-hazard regression models. In the forestplots, statistically significant results are displayed as filled points (P<0.001 corresponding to multiple testing correction) and non-significant results are displayed as hollow points. These results demonstrate that the 9 individual biomarkers are predictive of the risk for dying from prostate cancer in general population settings.

**Figure 3** shows the relation of baseline concentrations of 9 blood biomarkers to breast cancer survival, here analysed as mortality from breast cancer (ICD-10 diagnosis code C50 in death records) among females with a prevalent breast cancer diagnosis at the time of the blood sampling (ICD-10 diagnosis code C50 in hospital records). The biomarker concentrations are analysed in absolute concentrations scaled to standard deviations of the study population. The results are based on statistical analyses of 1 526 females from the UK Biobank with a prevalent breast cancer at the time of the blood sampling, out of whom 130 individuals died from the breast cancer during approximately 10 years of follow-up. The analyses were adjusted for age and UK Biobank assessment centre in Cox proportional-hazard regression models. In the forestplots, statistically significant results are displayed as filled points (P<0.001 corresponding to multiple testing correction) and non-significant results are displayed as hollow points. These results demonstrate that the 9 individual biomarkers are predictive of breast cancer survival among women with an existing form of breast cancer.

**Figure 4** shows the relation of baseline concentrations of 9 blood biomarkers to prostate cancer survival, here analysed as mortality from prostate cancer (ICD-10 diagnosis code C61 in death records) among males with a prevalent prostate cancer diagnosis at the time of the blood sampling (ICD-10 diagnosis code C61 in hospital records). The biomarker concentrations are analysed in absolute concentrations scaled to standard deviations of the study population. The results are based on statistical analyses of 584 males from the UK Biobank with a prevalent prostate cancer at the time of the blood sampling, out of whom 55 individuals died from the prostate cancer during approximately 10 years of follow-up. The analyses were adjusted for age and UK Biobank assessment centre in Cox proportional-hazard regression models. In the forestplots, statistically significant results are displayed as filled points (P<0.001 corresponding to multiple testing correction) and non-significant results are displayed as hollow points. These results demonstrate that the 9 individual biomarkers are predictive of prostate cancer survival among men with an existing form of prostate cancer.

### Illustrations of intended use: biomarker scores for risk prediction of death from breast cancer or prostate cancer

For illustration of intended applications related to the prediction of dying from breast cancer or prostate cancer, further epidemiological analyses are illustrated below. Results are shown for a biomarker score combining the 9 biomarkers featured in Figures 1-4. Similar results, albeit slightly weaker, are obtained with combinations of only two or three individual biomarkers.

**Figure 5a** shows the increase in the risk for breast cancer mortality (ICD-10 diagnosis code C50) in a general population screening setting (such as in conjuction with a mammography test) along with increasing levels of a biomarker risk score composed of a weighted sum of the 9 biomarkers. The risk increase is plotted in the form of Kaplan-Meier plots showing the cumulative mortality from breast cancer for selected quantiles of the plurality-biomarker-score.

**Figure 5b** shows the hazard ratios of the same plurality-biomarker score with breast cancer mortality risk when accounting for relevant risk factor characteristics of the study participants. The first panel demonstrates that the risk prediction works effectively for people at different ages at the time of blood sampling. The second panel shows that the magnitude of the hazard ratio is only modestly attenuated when accounting for body mass index and smoking status in the statistical modelling. The last panel demonstrates that the hazard ratios are substantially stronger for short term risk prediction.

**Figure 6a** shows the increase in the risk for prostate cancer mortality (ICD-10 diagnosis code C61) in a general population screening setting (such as in conjuction with a prostate-specific antigen (PSA) test) along with increasing levels of a biomarker risk score composed of a weighted sum of the 9 biomarkers. The risk increase is plotted in the form of Kaplan-Meier plots showing the cumulative mortality from prostate cancer for selected quantiles of the plurality-biomarker-score.

**Figure 6b** shows the hazard ratios of the same plurality-biomarker score with prostate cancer mortality risk when accounting for relevant risk factor characteristics of the study participants. The first panel demonstrates that the risk prediction works effectively for people at different ages at the time of blood sampling. The second panel shows that the magnitude of the hazard ratio is only modestly attenuated when accounting for body mass index and smoking status in the statistical modelling. The last panel demontrates that the hazard ratios are substantially stronger for short term risk prediction.

It is obvious to a person skilled in the art that with the advancement of technology, the basic idea may be implemented in various ways. The embodiments are thus not limited to the examples described above; instead they may vary within the scope of the claims.

The embodiments described hereinbefore may be used in any combination with each other. Several of the embodiments may be combined together to form a further embodiment. A method disclosed herein may comprise at least one of the embodiments described hereinbefore. It will be understood that the benefits and advantages described above may relate to one embodiment or may relate to several embodiments. The embodiments are not limited to those that solve any or all of the stated problems or those that have any or all of the stated benefits and advantages. It will further be understood that reference to 'an' item refers to one or more of those items. The term "comprising" is used in this specification to mean including the feature(s) or act(s) followed thereafter, without excluding the presence of one or more additional features or acts.

## Claims

1. A method for determining whether a subject is at risk of dying from breast cancer;
wherein the method comprises determining in a biological sample obtained from the subject a quantitative value of at least one biomarker of the following in the biological sample:
- glycoprotein acetyls,
- a ratio of docosahexaenoic acid to total fatty acids,
- a ratio of linoleic acid to total fatty acids,
- a ratio of monounsaturated fatty acids and/or of oleic acid to total fatty acids,
- a ratio of omega-3 fatty acids to total fatty acids,
- a ratio of omega-6 fatty acids to total fatty acids,
- a ratio of saturated fatty acids to total fatty acids,
- fatty acid degree of unsaturation,
- histidine; and
comparing the quantitative value(s) of the at least one biomarker to a control sample or to a control value;
wherein an increase or a decrease in the quantitative value(s) of the at least one biomarker, when compared to the control sample or to the control value, is/are indicative of the subject having an increased risk of dying from breast cancer;
wherein the at least one biomarker comprises or is glycoprotein acetyls; and
wherein the subject is a woman without known diagnosis of breast cancer.

2. The method according to claim 1, wherein the method comprises determining in the biological sample quantitative values of a plurality of biomarkers, such as two, three, four, five or more biomarkers.

3. The method according to claim 1 or 2, wherein the method comprises determining in the biological sample obtained from the subject a quantitative value of the following biomarkers:
- glycoprotein acetyls,
- at least one fatty acid measure(s) of the following: the ratio of docosahexaenoic acid to total fatty acids, the ratio of linoleic acid to total fatty acids, the ratio of monounsaturated fatty acids and/or of oleic acid to total fatty acids, the ratio of omega-3 fatty acids to total fatty acids, the ratio of omega-6 fatty acids to total fatty acids, the ratio of saturated fatty acids to total fatty acids, fatty acid degree of unsaturation; and
comparing the quantitative value(s) of the biomarkers to a control sample or to a control value(s);
wherein an increase or a decrease in the quantitative value(s) of the biomarkers, when compared to the control sample or to the control value, is/are indicative of the subject having an increased risk of dying from breast cancer.

4. The method according to any one of claims 1 - 3, wherein the method comprises determining in the biological sample obtained from the subject a quantitative value of the following biomarkers:
- glycoprotein acetyls,
- histidine,
- at least one fatty acid measure(s) of the following: the ratio of docosahexaenoic acid to total fatty acids, the ratio of linoleic acid to total fatty acids, the ratio of monounsaturated fatty acids and/or of oleic acid to total fatty acids, the ratio of omega-3 fatty acids to total fatty acids, the ratio of omega-6 fatty acids to total fatty acids, the ratio of saturated fatty acids to total fatty acids, fatty acid degree of unsaturation; and
comparing the quantitative value(s) of the biomarkers to a control sample or to a control value(s);
wherein an increase or a decrease in the quantitative value(s) of the biomarkers, when compared to the control sample or to the control value, is/are indicative of the subject having an increased risk of dying from breast cancer.

5. The method according to any one of claims 1 - 4, wherein the breast cancer comprises or is malignant neoplasm of breast (C50).

6. The method according to any one of claims 1 - 5, wherein the quantitative value of the at least one biomarker is/are measured using nuclear magnetic resonance spectroscopy.

7. The method according to any one of claims 1 - 6, wherein the method further comprises determining whether the subject is at risk of dying from breast cancer using a risk score, hazard ratio, odds ratio, and/or predicted absolute risk or relative risk calculated on the basis of the quantitative value(s) of at least one biomarker or of the plurality of the biomarkers.

8. The method according to any one of claims 1 - 7, wherein the control sample or control value represents women without known diagnosis of breast cancer, or women already diagnosed with breast cancer.

9. The method according to any one of claims 1 - 8, wherein the subject is a woman in a general population screening setting, such as a mammography.

10. The method according to any one of claims 1 - 9, wherein the method comprises determining in the biological sample obtained from the subject a quantitative value of the following biomarkers:
- glycoprotein acetyls,
- a ratio of docosahexaenoic acid to total fatty acids,
- a ratio of linoleic acid to total fatty acids,
- a ratio of monounsaturated fatty acids and/or of oleic acid to total fatty acids,
- a ratio of omega-3 fatty acids to total fatty acids,
- a ratio of omega-6 fatty acids to total fatty acids,
- a ratio of saturated fatty acids to total fatty acids,
- fatty acid degree of unsaturation,
- histidine; and
comparing the quantitative value(s) of the biomarkers to a control sample or to a control value(s);
wherein an increase or a decrease in the quantitative value(s) of the biomarkers, when compared to the control sample or to the control value, is/are indicative of the subject having an increased risk of dying from breast cancer.

## Patentansprüche

1. Verfahren zum Bestimmen, ob bei einer Person ein Risiko besteht, an Brustkrebs zu sterben;
wobei das Verfahren das Bestimmen eines quantitativen Werts mindestens eines der folgenden Biomarker in einer von der Person erhaltenen biologischen Probe umfasst:
• Glykoproteinacetyle,
• ein Verhältnis von Docosahexaensäure zu den Gesamtfettsäuren,
• ein Verhältnis von Linolsäure zu den Gesamtfettsäuren,
• ein Verhältnis von einfach ungesättigten Fettsäuren und/oder von Ölsäure zu den Gesamtfettsäuren,
• ein Verhältnis von Omega-3-Fettsäuren zu den Gesamtfettsäuren,
• ein Verhältnis von Omega-6-Fettsäuren zu den Gesamtfettsäuren,
• ein Verhältnis von gesättigten Fettsäuren zu den Gesamtfettsäuren,
• den Ungesättigtheitsgrad der Fettsäuren,
• Histidin; und
das Vergleichen des quantitativen Werts bzw. der quantitativen Werte des mindestens einen Biomarkers mit einer Kontrollprobe oder mit einem Kontrollwert;
wobei eine Zunahme oder eine Abnahme des quantitativen Werts bzw. der quantitativen Werte des mindestens einen Biomarkers bei einem Vergleich mit der Kontrollprobe oder dem Kontrollwert darauf hinweist, dass bei der Person ein erhöhtes Risiko besteht, an Brustkrebs zu sterben;
wobei der mindestens eine Biomarker Glykoproteinacetyle umfasst oder Glykoproteinacetyle darstellt; und
wobei die Person eine Frau ohne bekannte Diagnose von Brustkrebs ist.

2. Verfahren nach Anspruch 1, wobei das Verfahren das Bestimmen quantitativer Werte einer Mehrzahl von Biomarkern in der biologischen Probe umfasst, wie etwa von zwei, drei, vier, fünf oder mehr Biomarkern.

3. Verfahren nach Anspruch 1 oder 2, wobei das Verfahren das Bestimmen eines quantitativen Werts der folgenden Biomarker in der von der Person erhaltenen biologischen Probe umfasst:
• Glykoproteinacetyle,
• mindestens eine der folgenden Fettsäuremessgrößen: das Verhältnis von Docosahexaensäure zu den Gesamtfettsäuren, das Verhältnis von Linolsäure zu den Gesamtfettsäuren, das Verhältnis von einfach ungesättigten Fettsäuren und/oder von Ölsäure zu den Gesamtfettsäuren, das Verhältnis von Omega-3-Fettsäuren zu den Gesamtfettsäuren, das Verhältnis von Omega-6-Fettsäuren zu den Gesamtfettsäuren, das Verhältnis von gesättigten Fettsäuren zu den Gesamtfettsäuren, der Ungesättigtheitsgrad der Fettsäuren; und
das Vergleichen des quantitativen Werts bzw. der quantitativen Werte der Biomarker mit einer Kontrollprobe oder mit einem oder mehreren Kontrollwerten;
wobei eine Zunahme oder eine Abnahme des quantitativen Werts bzw. der quantitativen Werte der Biomarker bei einem Vergleich mit der Kontrollprobe oder dem Kontrollwert darauf hinweist, dass bei der Person ein erhöhtes Risiko besteht, an Brustkrebs zu sterben.

4. Verfahren nach einem der Ansprüche 1 bis 3, wobei das Verfahren das Bestimmen eines quantitativen Werts der folgenden Biomarker in der von der Person erhaltenen biologischen Probe umfasst:
• Glykoproteinacetyle,
• Histidin,
• mindestens eine der folgenden Fettsäuremessgrößen: das Verhältnis von Docosahexaensäure zu den Gesamtfettsäuren, das Verhältnis von Linolsäure zu den Gesamtfettsäuren, das Verhältnis von einfach ungesättigten Fettsäuren und/oder von Ölsäure zu den Gesamtfettsäuren, das Verhältnis von Omega-3-Fettsäuren zu den Gesamtfettsäuren, das Verhältnis von Omega-6-Fettsäuren zu den Gesamtfettsäuren, das Verhältnis von gesättigten Fettsäuren zu den Gesamtfettsäuren, der Ungesättigtheitsgrad der Fettsäuren; und
das Vergleichen des quantitativen Werts bzw. der quantitativen Werte der Biomarker mit einer Kontrollprobe oder mit einem oder mehreren Kontrollwerten;
wobei eine Zunahme oder eine Abnahme des quantitativen Werts bzw. der quantitativen Werte der Biomarker bei einem Vergleich mit der Kontrollprobe oder dem Kontrollwert darauf hinweist, dass bei der Person ein erhöhtes Risiko besteht, an Brustkrebs zu sterben.

5. Verfahren nach einem der Ansprüche 1 bis 4, wobei der Brustkrebs eine bösartige Neubildung der Brustdrüse (C50) umfasst oder darstellt.

6. Verfahren nach einem der Ansprüche 1 bis 5, wobei der quantitative Wert des mindestens einen Biomarkers mittels Kernmagnetresonanzspektroskopie gemessen wird.

7. Verfahren nach einem der Ansprüche 1 bis 6, wobei das Verfahren ferner das Bestimmen umfasst, ob bei der Person ein Risiko besteht, an Brustkrebs zu sterben, unter Verwendung eines Risikoscores, einer Hazard-Ratio, einer Odds-Ratio und/oder eines vorhergesagten absoluten Risikos oder relativen Risikos, berechnet auf Grundlage des quantitativen Werts bzw. der quantitativen Werte des mindestens einen Biomarkers oder der Mehrzahl der Biomarker.

8. Verfahren nach einem der Ansprüche 1 bis 7, wobei die Kontrollprobe oder der Kontrollwert Frauen ohne bekannte Diagnose von Brustkrebs oder Frauen, bei denen bereits Brustkrebs diagnostiziert wurde, repräsentiert.

9. Verfahren nach einem der Ansprüche 1 bis 8, wobei die Person eine Frau im Rahmen eines Screenings der Allgemeinbevölkerung, wie etwa einer Mammographie, ist.

10. Verfahren nach einem der Ansprüche 1 bis 9, wobei das Verfahren das Bestimmen eines quantitativen Werts der folgenden Biomarker in der von der Person erhaltenen biologischen Probe umfasst:
• Glykoproteinacetyle,
• ein Verhältnis von Docosahexaensäure zu den Gesamtfettsäuren,
• ein Verhältnis von Linolsäure zu den Gesamtfettsäuren,
• ein Verhältnis von einfach ungesättigten Fettsäuren und/oder von Ölsäure zu den Gesamtfettsäuren,
• ein Verhältnis von Omega-3-Fettsäuren zu den Gesamtfettsäuren,
• ein Verhältnis von Omega-6-Fettsäuren zu den Gesamtfettsäuren,
• ein Verhältnis von gesättigten Fettsäuren zu den Gesamtfettsäuren,
• den Ungesättigtheitsgrad der Fettsäuren,
• Histidin; und
das Vergleichen des quantitativen Werts bzw. der quantitativen Werte der Biomarker mit einer Kontrollprobe oder mit einem oder mehreren Kontrollwerten;
wobei eine Zunahme oder eine Abnahme des quantitativen Werts bzw. der quantitativen Werte der Biomarker bei einem Vergleich mit der Kontrollprobe oder dem Kontrollwert darauf hinweist, dass bei der Person ein erhöhtes Risiko besteht, an Brustkrebs zu sterben.

## Revendications

1. Procédé pour déterminer si un sujet risque de mourir à cause d'un cancer du sein ;
dans lequel le procédé comprend la détermination, dans un échantillon biologique obtenu à partir du sujet, d'une valeur quantitative d'au moins un biomarqueur parmi les suivants dans l'échantillon biologique :
- les acétyles de glycoprotéines,
- un rapport acide docosahexaénoïque sur acides gras totaux,
- un rapport acide linoléique sur acides gras totaux,
- un rapport acides gras mono-insaturés et/ou acide oléique sur acides gras totaux,
- un rapport acides gras oméga-3 sur acides gras totaux,
- un rapport acides gras oméga-6 sur acides gras totaux,
- un rapport acides gras saturés sur acides gras totaux,
- le degré d'insaturation des acides gras,
- l'histidine ; et
la comparaison de la ou des valeur(s) quantitative(s) de l'au moins un biomarqueur à un échantillon témoin ou à une valeur témoin ;
dans lequel une augmentation ou une diminution de la(des) valeur(s) quantitative(s) de l'au moins un biomarqueur, par rapport à l'échantillon témoin ou à la valeur témoin, indique que le sujet présente un risque accru de mourir à cause d'un cancer du sein ;
dans lequel l'au moins un biomarqueur comprend ou est des acétyles de glycoprotéines ; et
dans lequel le sujet est une femme sans diagnostic connu de cancer du sein.

2. Procédé selon la revendication 1, dans lequel le procédé comprend la détermination dans l'échantillon biologique de valeurs quantitatives d'une pluralité de biomarqueurs, telle que deux, trois, quatre, cinq ou plus de cinq biomarqueurs.

3. Procédé selon la revendication 1 ou la revendication 2, dans lequel le procédé comprend la détermination, dans l'échantillon biologique obtenu à partir du sujet, d'une valeur quantitative des biomarqueurs suivants :
- les acétyles de glycoprotéines,
- au moins une mesure d'acide gras parmi les suivantes : le rapport acide docosahexaénoïque sur acides gras totaux, le rapport acide linoléique sur acides gras totaux, le rapport acides gras mono-insaturés et/ou acide oléique sur acides gras totaux, le rapport acides gras oméga-3 sur acides gras totaux, le rapport acides gras oméga-6 sur acides gras totaux, le rapport acides gras saturés sur acides gras totaux, le degré d'insaturation des acides gras ; et
la comparaison de la ou des valeur(s) quantitative(s) des biomarqueurs à un échantillon témoin ou à une ou plusieurs valeur(s) témoin(s) ;
dans lequel une augmentation ou une diminution de la(des) valeur(s) quantitative(s) des biomarqueurs, par rapport à l'échantillon témoin ou à la valeur témoin, indique que le sujet présente un risque accru de mourir à cause d'un cancer du sein.

4. Procédé selon l'une quelconque des revendications 1 à 3, dans lequel le procédé comprend la détermination, dans l'échantillon biologique obtenu à partir du sujet, d'une valeur quantitative des biomarqueurs suivants :
- les acétyles de glycoprotéines,
- l'histidine,
- au moins une mesure d'acide gras parmi les suivantes : le rapport acide docosahexaénoïque sur acides gras totaux, le rapport acide linoléique sur acides gras totaux, le rapport acides gras mono-insaturés et/ou acide oléique sur acides gras totaux, le rapport acides gras oméga-3 sur acides gras totaux, le rapport acides gras oméga-6 sur acides gras totaux, le rapport acides gras saturés sur acides gras totaux, le degré d'insaturation des acides gras ; et
la comparaison de la ou des valeur(s) quantitative(s) des biomarqueurs à un échantillon témoin ou à une ou plusieurs valeur(s) témoin(s) ;
dans lequel une augmentation ou une diminution de la(des) valeur(s) quantitative(s) des biomarqueurs, par rapport à l'échantillon témoin ou à la valeur témoin, indique que le sujet présente un risque accru de mourir à cause d'un cancer du sein.

5. Procédé selon l'une quelconque des revendications 1 à 4, dans lequel le cancer du sein comprend ou est un néoplasme malin du sein (C50).

6. Procédé selon l'une quelconque des revendications 1 à 5, dans lequel la valeur quantitative de l'au moins un biomarqueur est/sont mesurée(s) en utilisant une spectroscopie par résonance magnétique nucléaire.

7. Procédé selon l'une quelconque des revendications 1 à 6, dans lequel le procédé comprend en outre la détermination si le sujet risque de mourir à cause d'un cancer du sein en utilisant un score de risque, un rapport de risque, un rapport de cotes et/ou un risque absolu ou un risque relatif prédit calculé sur la base de la(des) valeur(s) quantitative(s) d'au moins un biomarqueur ou de la pluralité des biomarqueurs.

8. Procédé selon l'une quelconque des revendications 1 à 7, dans lequel l'échantillon témoin ou la valeur témoin représente des femmes sans diagnostic connu de cancer du sein, ou des femmes ayant déjà reçu un diagnostic de cancer du sein.

9. Procédé selon l'une quelconque des revendications 1 à 8, dans lequel le sujet est une femme dans un contexte de dépistage de la population générale, tel qu'une mammographie.

10. Procédé selon l'une quelconque des revendications 1 à 9, dans lequel le procédé comprend la détermination, dans l'échantillon biologique obtenu à partir du sujet, d'une valeur quantitative des biomarqueurs suivants :
- les acétyles de glycoprotéines,
- un rapport acide docosahexaénoïque sur acides gras totaux,
- un rapport acide linoléique sur acides gras totaux,
- un rapport acides gras mono-insaturés et/ou acide oléique sur acides gras totaux,
- un rapport acides gras oméga-3 sur acides gras totaux,
- un rapport acides gras oméga-6 sur acides gras totaux,
- un rapport acides gras saturés sur acides gras totaux,
- le degré d'insaturation des acides gras,
- l'histidine ; et
la comparaison de la ou des valeur(s) quantitative(s) des biomarqueurs à un échantillon témoin ou à une ou plusieurs valeur(s) témoin(s) ;
dans lequel une augmentation ou une diminution de la(des) valeur(s) quantitative(s) des biomarqueurs, par rapport à l'échantillon témoin ou à la valeur témoin, indique que le sujet présente un risque accru de mourir à cause d'un cancer du sein.
